# FASCICULE DE BREVET EUROPEEN

(11) **EP 0 539 281 B1**
(45) Date de publication et mention de la délivrance du brevet: **30.12.1998**
(21) Numéro de dépôt: 92402862.4
(22) Date de dépôt: 20.10.1992
(51) Int. Cl.: C07D 207/09, C07D 211/58, C07C 235/66, A61K 31/40, A61K 31/445, C07D 295/13

(54) **Dérivés de naphtamides, leur procédé de préparation et leur application dans le domaine thérapeutique**
Naphthamide, Verfahren zur Herstellung und ihre therapeutische Anwendung
Naphthamides, process for their preparation and their application in the therapeutical field

(30) Priorité: 23.10.1991 FR 9113103
(43) Date de publication de la demande: 28.04.1993
(73) Titulaire: INSTITUT NATIONAL DE LA SANTE ET DE LA RECHERCHE MEDICALE (INSERM), 75654 Paris Cédex 13 (FR); SOCIETE CIVILE BIOPROJET, F-75003 Paris (FR)
(72) Inventeur: Rognan, Didier, F-68300 Saint-Louis (FR); Mann, André, F-67540 Ostwald (FR); Wermuth, Camille-Georges, F-67100 Strasbourg (FR); Martres, Marie-Pascale, F-75015 Paris (FR); Giros, Bruno, F-92320 Chatillon (FR); Sokoloff, Pierre, F-95130 Le Plessis Bouchard (FR); Schwartz, Jean-Charles, F-75014 Paris (FR); Lecomte, Jeanne-Marie, F-75003 Paris (FR); Garrido, Fabrice, F-67100 Strasbourg (FR)
(74) Mandataire: Bernasconi, Jean

(56) Documents cités:
- EP-A- 393 838
- GB-A- 2 176 785
- US-A- 4 540 814
- JOURNAL OF MEDICINAL CHEMISTRY. vol. 33, no. 1, 1990, WASHINGTON US pages 171 - 178 MURPHY R. A. ET. AL. 'Synthesis and Characterisation of Iodobenzamide Analogues: Potential D-2 Dopamine Receptor Imaging Agents'
- JOURNAL OF MEDICINAL CHEMISTRY. vol. 31, no. 8, 1988, WASHINGTON US pages 1548 - 1558 MONKOVIC I. ET. AL. 'Substituted Benzamides'
- CHEMICAL ABSTRACTS, vol. 74, 1971, Columbus, Ohio, US; abstract no. 53340, & FR-A-1 587 740 (TECNIFAX) 27 Mars 1970
- CHEMICAL ABSTRACTS, vol. 72, 1970, Columbus, Ohio, US; abstract no. 43286, & FR-A-1 558 147 (KONISHIROKU) 21 Février 1969

## Description

La présente invention a trait à de nouveaux dérivés de naphtamides, à leur procédé de préparation et à leur application dans le domaine thérapeutique.

De nombreux dérivés de benzamides, notamment de l'ortho-méthoxybenzamide, sont connus et utilisés pour leurs activités sur le système nerveux central, en particulier pour leurs propriétés neuroleptiques (Justin-Besançon et al., C.R. Acad. Sci. Paris, 1964, 265 : 1253 - 1254 ; Jenner et Marsden, Life Sci. 1979, 25 : 479-486); GB-A-2 176 785).

D'autres benzamides substitués, modifiés notamment au niveau du groupe ortho-méthoxy, sont dénués d'activité antagoniste dopaminergique (Monkovic I. et al., J. Med. Chem., 1988, 31, 1548-1558).

Par contre, les dérivés de 1-méthoxy-naphtamides correspondants n'ont pas été décrits dans la littérature. Or, le demandeur a découvert que, de façon surprenante, les dérivés de 1-méthoxy-naphtamides de formule générale (I), donnée ci-dessous, sont caractérisés par une affinité plus forte pour les récepteurs dopaminergiques, que celle des benzamides jusqu'ici connus. De plus, ces nouveaux dérivés présentent un certain pouvoir discriminant entre les récepteurs de la sous-classe D₂ et ceux de la sous-classe D₃ (Sokoloff et al. Nature 1990, 347 : 146 - 151). L'affinité préférentielle pour les récepteurs D₃, présentée par les nouveaux dérivés de naphtamides conformes à l'invention, autorise une thérapeutique neuroleptique exempte des syndromes extrapyramidaux.

On connaît du document US-A-4 540 814, des composés de type 1,4-diméthoxy-2-naphtamides, notamment le 1,4-diméthoxy-N-(2-diméthylaminoéthyl)-2-naphtamide, utiles comme agents anti-épileptiques

Les documents EP-A-0 393 838 et J. Med Chem, 1990, 33, 171-178, décrivent par ailleurs, des dérivés 2-méthoxy-3-naphtamides utiles comme agents d'imagerie du récepteur D2 de la dopamine et comme agents de diagnostic.

Les nouveaux dérivés de naphtamides conformes à l'invention sont caractérisés en ce qu'ils répondent à la formule générale (I), 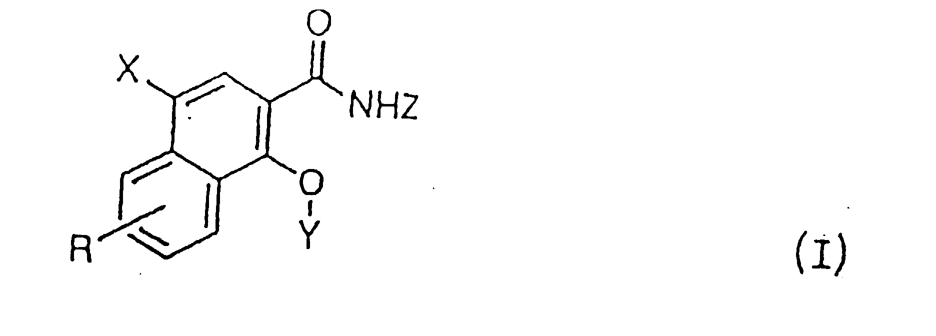 où
X : représente soit un atome d'hydrogène, soit un atome de chlore ou de brome, soit un groupement amino- ou amino-alkyle, un groupe méthane sulfonylamino, un groupe thiocyanate, alkylthio, alkylsulfinyle ou alkylsulfonyle, soit un groupe méthoxy, soit un groupe nitro, soit un groupe cyano,
Y : représente un reste alkyle ou alcényle
Z :représente les restes 2-méthyl N-alkylpyrrolidine, 2-éthyl - N,N - diéthylamine, 2-éthyl morpholine, 2-éthyl-N,N-dibutylamine, 4-N-butyl pipéridine, 4-N-benzyl pipéridine, 2-éthylpyrrolidine.
R : un hydrogène ou un substituant OCH₃.

Selon un mode de réalisation les composés peuvent répondre à la formule 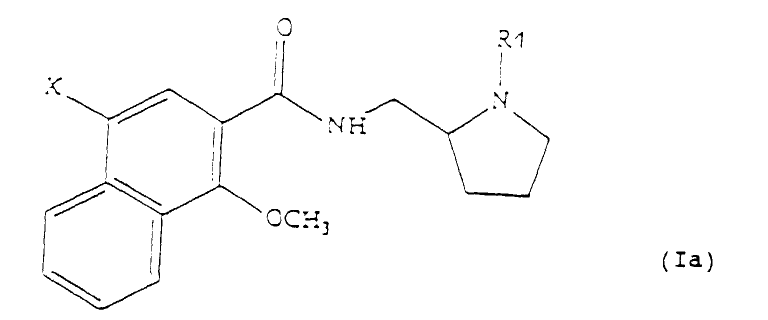 où
X représente un atome d'hydrogène, un atome d'halogène tel que le chlore ou le brome, un groupe amino ou aminoalkyle, un groupe + méthane sulfonylamino, un groupe alcoxy inférieur tel qu'un groupe méthoxy, un groupe nitro ou encore un groupe cyano,
R1 représente un groupe alkyle inférieur, un groupe cycloalkyle-alkyle inférieur, un groupe aralkyle tel qu'un groupe phénylalkyle ou encore le groupe allyle.

La présente invention concerne également les sels d'addition que forment les composés de formule (I,Ia) avec les acides physiologiquement acceptables tels que les acides chlorhydrique, sulfurique, nitrique, maléique, etc.

Par alkyle inférieur, on entend un radical alkyle linéaire ou ramifié contenant 1 à 6 atomes de carbone et plus particulièrement les radicaux méthyle, éthyle, propyle et butyle.

Les composés préférés de l'invention sont plus particulièrement ceux qui répondent à la formule générale (I, Ia) dans laquelle X représente un atome d'hydrogène,un atome de chlore ou de fluor, un groupe amino, nitro, cyano ou encore un groupe méthoxy ; et R1 représente un radical éthyle, propyle ou butyle, le groupe benzyle ou un groupe cycloalkyle-alkyle inférieur tel que le groupe cyclopropyle méthyle, ou encore le groupe allyle.

Les composés de formule (I) conformes à l'invention présentent un atome de carbone asymétrique et peuvent donc se présenter sous forme de racémates ou d'isomères optiques, lesquels font également partie de l'invention.

Parmi les composés plus particulièrement préférés, on peut citer les composés suivants :
- le N -[(N 1-butyl-2-pyrrolidinyl)-méthyl]-1-méthoxy-4-bromo-2-naphtamide, éventuellement sous forme de chlorhydrate,
- le N-[N(allyl-2-pyrrolidinyl)-méthyl]-1-méthoxy-4-cyano-2-naphtamide,
- le N-[(N-éthyl-2-pyrrolidinyl)-méthyl]-1-méthoxy -2-naphtamide,
- le 4-amino-N-[(N-éthyl-2-pyrrolidinyl)-méthyl]-1-méthoxy-2-naphtamide, éventuellement sous forme de dichlorhydrate,
- le 4-nitro-N-[(N-éthyl-2-pyrrolidinyl)-méthyl]-1-méthoxy-2-naphtamide,
- le N-[(N-éthyl-2-pyrrolidinyl)-méthyl]-1,4-diméthoxy-2-naphtamide, éventuellement sous forme de chlorhydrate,
- le 4-chloro-N-[(N-éthyl-2-pyrrolidinyl)-méthyl] -1-méthoxy-2-naphtamide, éventuellement sous forme de chlorhydrate,
- le 4-bromo-N-[(N-éthyl-2-pyrrolidinyl)-méthyl] -1-méthoxy-2-naphtamide, éventuellement sous forme de chlorhydrate,
- le 4-méthane sulfonylamino-N-[(N-éthyl-2-pyrrolidinyl)-méthyl]-1-méthoxy-2-naphtamide, éventuellement sous forme de chlorhydrate,
- le N-[(N-benzyl-2-pyrrolidinyl)-méthyl]-1-méthoxy-2-naphtamide, éventuellement sous forme de chlorhydrate,
- le 4-nitro-N-[(N-benzyl-2-pyrrolidinyl)-méthyl] -1-méthoxy-2-naphtamide,
- le 4-chloro-N-[(N-benzyl-2-pyrrolidinyl)-méthyl] -1-méthoxy-2-naphtamide,
- le 4-chloro-N-[(N-propyl-2-pyrrolidinyl)-méthyl] -1-méthoxy-2-naphtamide,
- le 4-bromo-N-[(N-cyclopropylméthyl-2-pyrrolidinyl) -méthyl]-1- méthoxy-2-naphtamide,
- le 4-chloro-N-[(N-allyl-2-pyrrolidinyl)-méthyl] -1-méthoxy-2-naphtamide,
- le 4-nitro-N-[(N-allyl-2-pyrrolidinyl)-méthyl] -1-méthoxy-2-naphtamide,
- le 4-cyano-N-[(N-éthyl-2-pyrrolidinyl)-méthyl] -1-méthoxy-2-naphtamide.
- le 4-cyano-N-[(N(3-butényl)-2-pyrrolidinyl)-méthyl]-1-méthoxy-2-naphtamide
- le 4-cyano-N-[(N-pentyl-2-pyrrolidinyl)-méthyl]-1-méthoxy-2-naphtamide
- le 4-cyano-N-[(N(4-pentényl)-2-pyrrolidinyl)-méthyl]-1-méthoxy-2-naphtamide
- le Chlorhydrate du 1,5-diméthoxy-N[(N-éthyl-2-pyrrolidinyl)-méthyl]-2-naphtamide
- le Chlorhydrate du 1,5-diméthoxy-N[(N-butyl-2-pyrrolidinyl)-méthyl]-2-naphtamide
- le 4-Bromo-N[(N,N-diéthylamino)-éthyl]-1-méthoxy-2-naphtamide
- le 4-cyano-N-[(N-butyl-2-pyrrolidinyl)-méthyl]-1-méthoxy-2-naphtamide
- le 4-Bromo-N[(morpholino)éthyl]-1-méthoxy-2-naphtamide
- le 4-Bromo-N[(N-butyl)-4'-piperidinyl]-1-méthoxy-2-naphtamide
- le 4-Bromo-N[(N-benzyl)-4'piperidinyl]-1-methoxy-2 naphtamide
- le 4-Nitro-N[(morpholino)-éthyl]-1-méthoxy-2-naphtamide
- le Chlorhydrate du 4-nitro-N[(N-butyl-2-pyrrolidinyl) -méthyl]-1,5-diméthoxy-2-naphtamide
- le 4-Nitro-N[N(butyl)-4'piperidinyl]-1-méthoxy-2-naphtamide
- le 4-Nitro-N[N,N-diéthylamino)-éthyl]-1-méthoxy-2-naphtamide
- le Chlorhydrate de la 4-bromo-N[N,N-dibutylamino)-éthyl]-1-méthoxy-2-naphtamide
- le 4-Bromo-N[(N-éthyl-2-pyrrolidinyl)-méthyl]-1-butoxy-2-naphtamide
- le 4-Nitro-N[(N-éthyl-2-pyrrolidinyl)-méthyl]-1,5-dinitro-2-naphtamide
- le 4-Bromo-N[(N-butyl)-4'piperidinyl]-1-allyloxy-2-naphtamide
- le Chlorhydrate du 4-bromo-N[(morpholinoéthyl)-1-allyloxy-2-naphtamide
- le Chlorhydrate du 1,4 diméthoxy-N[(N-butyl)-4'piperidinyl]-2-naphtamide
- le Chlorhydrate du 4-bromo-N[(morpholino)éthyl)-1-butoxy]-2-naphtamide
- le Chlorhydrate du 4-bromo-N[(N-éthyl-2-pyrrolidinyl) -méthyl]-1-cyclopentyl-oxy-2-naphtamide
- le Chlorhydrate du 4-nitro-N[(N,N-dibutylamino)éthyl] -1-méthoxy-2-naphtamide
- le 4-Bromo-N[(pyrrolidinyl éthyl)]-1-méthoxy-2-naphtamide
- le Chlorhydrate du 4-cyano-N[(N,N-dibutylamino)éthyl] -1-méthoxy-2-naphtamide

Les composés de formule (I) conformes à l'invention sont obtenus par des procédés de type connu.

La partie acide (1-méthoxy-4-X-naphtoïque), convenablement substituée, est transformée en anhydride mixte par le chloroformiate d'éthyle dans l'acétone ou tout autre solvant, en milieu basique et mis en réaction avec la pyrrolidine souhaitée, comme indiqué sur le shéma réactionnel :

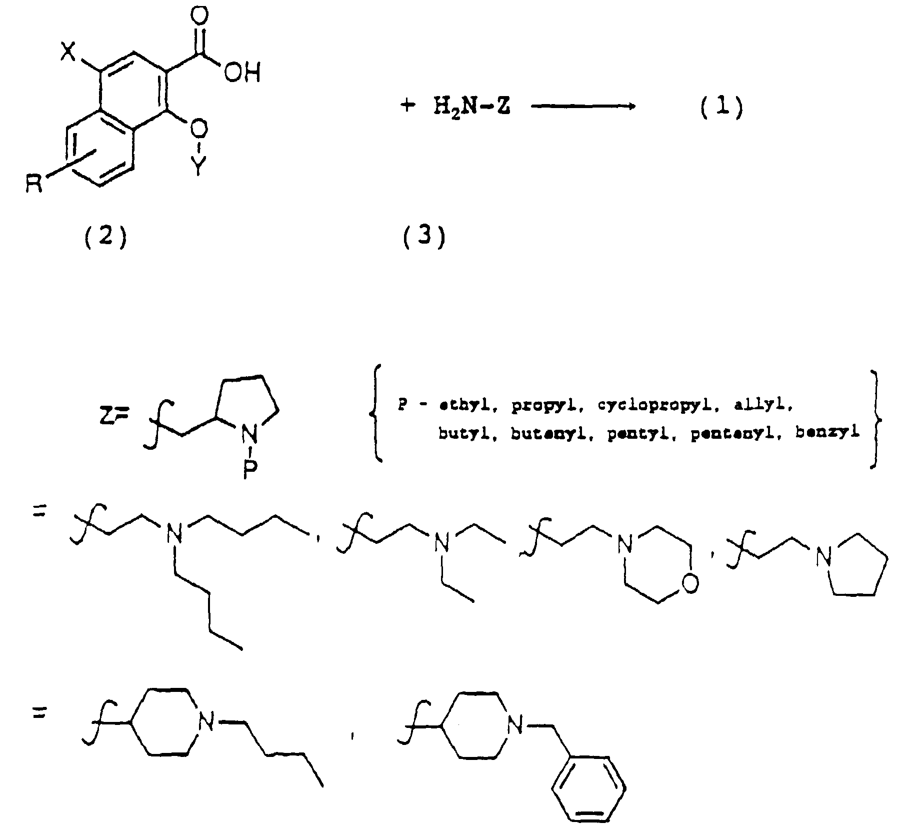

D'autres méthodes d'activation de la fonction carboxylique peuvent également être utilisées, en fait toutes les méthodes de préparation d'amide conviennent, y compris l'emploi du chlorure d'acide.

Pour R = OCH₃, le produit de départ a été de l'acide l-hydroxy-5-méthoxy-2-naphtoïque, décrit dans la littérature (J. Chem. Soc., 1937, 937).

Le groupement X est introduit sur l'acide naphtoïque, soit par du brome (X = Br), soit par nitration (X = NO2), et réduction (X = NH₂), et acylation (X = NHSO₂CH₃), soit par une réaction de Sandmeyer (X = Cl, CN).

Pour Y = alkyl, la matière première a été l'acide 1-hydroxy-4-X-naphtoïque qui a été alkylé sur l'atome d'oxygène en milieu basique par un haloqénure d'alkyle.

Pour X = OCH₃, la matière première a été l'acide 1,4-diméthoxy-2-naphtoïque. D'autre part, les différentes amines utilisées pour l'obtention d'amide ont été obtenues par les voies énumérées ci-dessous :
- A partir de N-éthyl-2-aminométhyl-pyrrolidine, 2-aminoéthyl N,N-diéthylamine, 2-amino éthylmorpholine, 2-aminoéthyl N,N-dibutylamine, 2-amino-éthyl pyrrolidine, qui sont commerciales.
- A partir de N-benzyl-pyrrolidine, selon les indications de Takashi et Masashi, DE-A-1.941.536 (5 mars 1970).
- A partir de butyrolactone et d'une amine (propylamine, butylamine et cyclopropylamine), selon les indications de Kaplan, DE-A- 2.556.457 (24 juin 1976).
- A partir de N-tert-butoxycarbonyl-aminométhyl-N-benzylpyrrolidine (V), suivi d'une débenzylation, d'une alkylation par le bromure d'allyle, par le bromo 1-butene , ou le 5-bromo-1 pentène et d'une déprotection pour obtenir la N-allyl-2-(ou N-butenyl ou N-pentenyl) aminométhyl-pyrrolidine (VI). 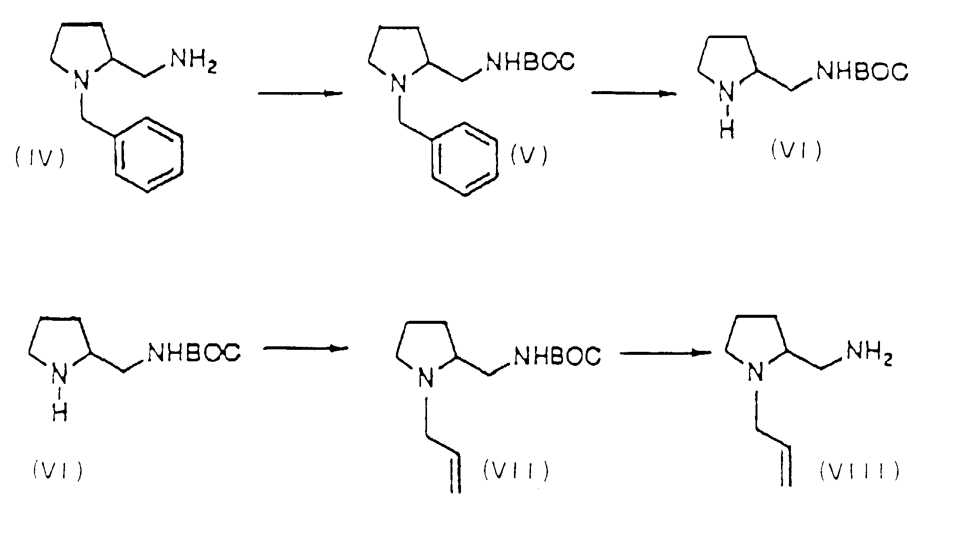
- A partir de N-benzyl-4(N-tert butoxycarboxyl) aminopipéridine par débenzylation suivie ,d'une alkylation par un halogénure d'alkyle et par une déprotection (voir schéma) pour obtenir l'amine XII.

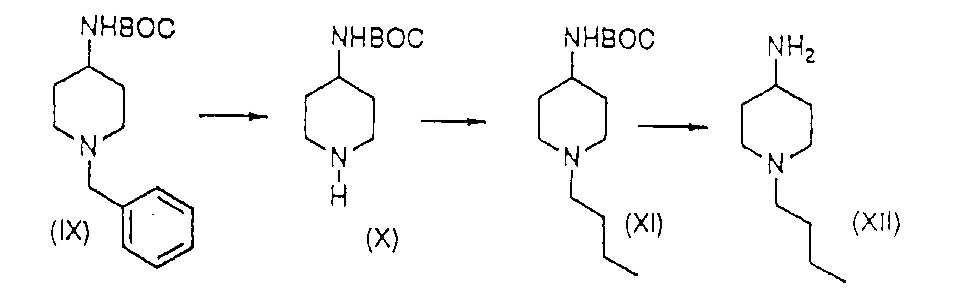

Ainsi les composés de formule (Ia) peuvent être obtenus selon le schéma réactionnel suivant :

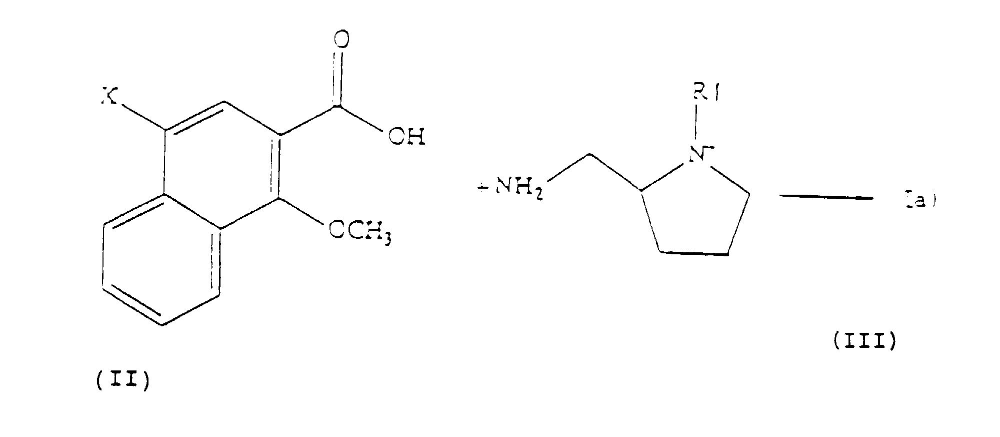

D'autres dérivés peuvent être préparés par des procédés nouveaux, en particulier ceux dans lesquels R1 représente un groupe allyle.

La présente invention englobe donc également le procédé de préparation de tels dérivés utilisés comme produits de départ dans la préparation des dérivés naphtamides de formule (Ia) conformes à la présente invention.

Le procédé de préparation de ces dérivés est caractérisé en ce que, partant de la 2-aminométhyl-N-pyrrolidine dont l'atome d'azote hétérocyclique porte un radical aralkyle tel que le benzyle (composé IV), il comporte les étapes :
- de protection de l'amine primaire par exemple sous forme de carbamate (composé V),
- de débenzylation de l'azote hétérocyclique,
- d'alkylation de l'azote hétérocyclique par un halogénure d'alkyle tel qu'un bromure d'alkyle,
- puis de déprotection de l'amine primaire, par exemple du carbamate (composé VI).

Il va être donné ci-dessous, à titre non limitatif, quelques exemples illustrant la réalisation de l'invention.

### Exemple 1 : Préparation du chlorhydrate du N-[(N-1-butyl-2-pyrrolidinyl)-méthyl]-1-méthoxy-4-bromo-2-naphtamide.

### 1. Préparation de l'ester méthylique de l'acide 1-hydroxy-4-bromo-2-naphtoïque.

On introduit dans un ballon de 100 ml 1,7 g (8,4 mmol) de l'ester méthylique de l'acide 1-hydroxy-2-naphtoïque et on ajoute goutte à goutte 0,54 ml (10 mmol) d'une solution de brome dans de l'acide acétique. On agite pendant 30 mn, à la température ambiante. On dilue avec de l'eau, on filtre le précipité formé, on lave avec de l'eau et on récupère 2,26 g du composé du titre - F 113°C (rendement = 95%).

### 2. Préparation de l'ester méthylique de l'acide 1-méthoxy-4-bromo-2-naphtoïque.

On chauffe à reflux pendant une nuit le mélange constitué de l'ester méthylique de l'acide 1-hydroxy-4-bromo-2-naphtoïque (2,26 g, 8 mmol), de carbonate de potassium (1,6 g, 0,012 mol), de diméthylsulfate (1,26 ml, 0,013 mol), le tout dans de l'acétone (50 ml). On filtre le précipité et on évapore l'acétone. On reprend le résidu dans de l'éther, on lave avec de l'eau, on séche et on évapore pour obtenir 1,9 g du composé du titre - F 80°C (rendement = 80%).

### 3. Préparation de l'acide 1-méthoxy-4-bromo-2-naphtoïque.

On chauffe à reflux pendant 48h, 1,8 g (6 mmol) de l'ester méthylique de l'acide 1-méthoxy-4-bromo-2-naphtoïque obtenu sous 2 dans 20 ml de méthanol en présence de 0,92 g (1,5 éq) de bicarbonate de potassium. On évapore le solvant, on reprend le résidu dans de l'eau et on extrait les neutres à l'éther. On acidifie ensuite la phase aqueuse avec de l'acide chlorhydrique dilué. On extrait avec de l'acétate d'éthyle, on lave avec de l'eau, on sèche et on concentre pour obtenir 1,63 g de l'acide du titre - F 197°C (rendement = 95%).

### 4. Préparation du chlorhydrate du N-[(N-1-butyl-2-pyrrolidinyl)-méthyl]-1-méthoxy-4-bromo-2-naphtamide

Dans un ballon de 25 ml, on introduit 281 mg (1 mmol) de l'acide 1-méthoxy-4-bromo-2-naphtoïque obtenu sous 3, 0,17 ml (1,2 mmol) de triéthylamine et 5 ml d'acétone. On refroidit à -15°C par l'intermédiaire d'un bain de glace. On ajoute goutte à goutte, 0,1 ml (1,05 mmol) de chloroformiate d'éthyle en solution dans 2 ml d'acétone. On agite 30 mn à - 15°C et on ajoute 164 mg (1,05 mmol) de la 2-aminométhyl-N-butyl-pyrrolidine. On laisse revenir le milieu réactionnel à la température ambiante, on filtre le précipité obtenu et on concentre sous vide. On reprend le résidu dans de l'eau, puis on extrait à l'acétate d'éthyle (2 x 30 ml). On lave avec de l'eau, on sèche la phase organique (Na₂S0₄) et on concentre celle-ci. Le résidu est chromatographié sur une colonne de silice en utilisant comme éluant un mélange acétate d'éthyle-méthanol (85 : 15), pour récupérer 310 mg du composé du titre - F 54°C

| | | | | |
|---|---|---|---|---|
| (rendement = 74%). | Cal. | C% 60,14 ; | H% 6,49 ; | N% 6,68 |
| | Tr. | C% 60,38 ; | H% 6,54 ; | N% 6,62. |

### Exemple 2 : Préparation du N-[n(allyl-2-pyrrolidinyl)-méthyl]-1-méthoxy-4-cyano-2-naphtamide.

### 1. Préparation de l'ester méthylique de l'acide 1-méthoxy -4-cyano-2-naphtoïque.

On dissout 1,16 g (432 mmol) de l'ester méthylique de l'acide 1-méthoxy-4-amino-naphtoïque dans 10 ml de HCl 3N. On ajoute à 0°C goutte à goutte 3 ml d'une solution aqueuse de 0,415 g (1,2 éq) de nitrite de sodium. On agite le milieu réactionnel 30 mn à 0°C. On neutralise ensuite à pH 4 avec de l'acétate de sodium. On prépare, en même temps, une solution de KCN (0,7 g) dans l'eau (6 ml), on ajoute du CuCN (0,43 g), on maintient cette solution à 0°C et on ajoute goutte à goutte la solution de diazonium. On abandonne la réaction 2 h à 5°C, puis on laisse monter la température. On filtre le précipité obtenu et on purifie par chromatographie, en utilisant comme éluant un mélange acétate d'éthyle-hexane, pour récupérer 500 mg du composé du titre - F 92°C, IR 2200 cm-¹ (rendement = 70%).

### 2. Préparation de l'acide 1-méthoxy-4-cyano-2-naphtoïque.

On porte à reflux pendant 24 h dans 25 ml de méthanol l'ester méthylique de l'acide 1-méthoxy-4-cyano-2-naphtoïque obtenu sous 1 (500 mg, 2 mmol) et du bicarbonate de potassium (0,3 g, 1,5 éq). On évapore le méthanol, on reprend dans de l'eau et on acidifie avec de l'acide chlorhydrique dilué. On extrait avec de l'acétate d'éthyle, on lave la phase organique avec de l'eau, on sèche et on évapore. On récupère 420 mg du composé du titre sous forme d'un solide -F 186°C, IR 2200 cm⁻¹ (rendement = 92%).

### 3. Préparation de la N-tert-butoxy-carbonyl-aminométhyl-N'-benzyl-pyrrolidine (composé V).

Dans un ballon de 250 ml, on introduit 3,42 g (18 mmol) de 2-aminométhyl-N-benzylpyrrolidine (composé IV) dissoute dans 100 ml de tétrahydrofurane (THF). On ajoute goutte à goutte une solution de di tert-butyl dicarbonate en solution dans 30 ml de THF. On agite à la température ambiante pendant la nuit. On concentre sous vide, on reprend le résidu formé dans de l'eau, puis on extrait avec de l'éther. On sèche la phase organique, puis on évapore. On purifie le résidu par chromatographie sur une colonne de silice en utilisant, comme éluant, un mélange hexane-éther, pour récupérer 5 g du composé V du titre (rendement = 95%).

### 4. Préparation de la N-tert-butoxycarbonyl-2-amino-méthylpyrrolidine (composé VI).

On porte à reflux pendant 2h, un mélange du composé V (5 g, 0,017 mol) dans du méthanol (125 ml) en présence de Pd/C à 10% (540 mg) et formiate d'ammonium (5,69 g). On refroidit et on filtre le catalyseur. On évapore le méthanol et on reprend dans une solution saturée en carbonate de potassium. On extrait ensuite à l'éther, on sèche et on concentre sous vide pour obtenir 3,7 g du composé V du titre (rendement = 99%) qui est utilisé tel quel pour la réaction suivante.

### 5. Préparation de la N-tert-butoxycarbonyl-2-aminométhyl-N'-allylpyrrolidine (composé VII).

On chauffe à reflux pendant 10h un mélange du composé (VI) (3,68 g, 0,017 mol), de bicarbonate de sodium (1,51 g, 0,018 mol) et de bromure d'allyle (1,55 g, 0,018 mol), de l'éthanol (170 ml). On évapore et on reprend par de l'eau. On extrait ensuite à l'éther, on sèche, on évapore et on purifie le résidu sur une colonne de silice en utilisant, comme éluant, de l'acétate d'éthyle, pour récupérer 2,27 g d'une huile (composé VII, rendement = 55%).

### 6. Préparation de la 2-aminométhyl-N-allylpyrrolidine (composé VIII).

On agite à température ambiante pendant 1 h, un mélange du composé (VII) (1 g, 4,16 mmol), d'acide acétique (33 ml) et d'acide chlorhydrique concentré (11 ml). On évapore, on reprend le résidu plusieurs fois avec de l'isopropanol, puis on évapore sous vide. On dissout ensuite le résidu dans du méthanol et on ajoute de la triéthylamine (0,58 ml, 4,16 mmol). On évapore le méthanol, on reprend par de l'éther, on filtre, on sèche et on concentre pour obtenir (590 mg) du composé du titre (rendement = 90%).

### 7. Préparation du N-[(allyl-2-pyrrolidinyl)méthyl]-1-méthoxy-4-cyano-2-naphtamide.

Dans un tricol de 25 ml sous argon, on introduit l'acide 1-méthoxy-4-cyano-2-naphtoïque (360 mg, 1, 58 mmol) obtenu sous 2 dans de l'acétone (8 ml). On ajoute de la triéthylamine et on refroidit à -15°C. On ajoute ensuite goutte à goutte du chloroformiate d'éthyle (0,26 ml, 1,2 éq) en solution dans de l'acétone (3 ml). On agite 30 mn à -15°C. On ajoute de la triéthylamine (0,26 ml), puis goutte à goutte du chlorhydrate de la 2-aminométhyl-N-allylpyrrolidine (composé VIII) en solution dans l'acétone (3 ml). On laisse évoluer le milieu réactionnel vers la température ambiante. On filtre le précipité obtenu et on évapore l'acétone. On reprend dans de l'eau, on extrait avec de l'acétate d'éthyle, on sèche et on évapore. On purifie le résidu par chromatographie sur une colonne de silice en utilisant, comme éluant, un mélange acétate d'éthyle-méthanol 90-10, pour obtenir 210 mg du composé du titre - F 93°C (rendement 38%).

| Analyse : C₂₁ H₂₃ N₃ O₂ | | | |
|---|---|---|---|
| Calc. | C% 72,02 ; | H% 6,80 ; | N% 12,09 |
| Trouvé | C% 72, 18 ; | H% 6,63 ; | N% 12,02. |

### Exemple 3 : 4-Cyano-N[N-butyl-2-pyrrolidinyl)-méthyl]-1-methoxy-2-naphtamide

Dans un tricol de 25 ml, sous argon, introduire l'acide 1-methoxy-4-cyano-2-naphtoïque (227 µg, 1 mmol) dans de l'acétone (5 ml). Ajouter de la triéthylamine (0,17 ml) et refroidir à -15°C. Ajouter goutte à goutte du chloroformiate d'éthyle (0,1 ml) dissout dans l'acétone (2 ml). Laisser agiter 30 min à -15°C. Ajouter la 2-méthylamino-N-butyl pyrrolidine (164 mg, 1,05 mmol) dissout dans de l'acétone (2 ml). Abandonner à température ambiante. Filtrer le chlorhydrate de triéthylamine formé, évaporer l'acétone, reprendre le résidu dans de l'eau, extraire avec de l'acétate d'éthyle. Laver la phase acétate d'éthyle avec de l'eau. Sécher, évaporer, obtenir une huile qui cristallise. Purifier par chromatographie sur colonne de silice (AcOEt-MeOH : 80-20). Recristalliser dans l'hexane pour obtenir le composé titre (210 mg, 57 %)
Analyse C₂₂H₂₇N₃O₂
Trouvé C % 72,37 ; H % 6,63 ; N % 12,00 ; Calc. C % 72,29 ; H % 7,47 ; N % 11,49.

### Exemples 4 à 42 :

Dans la liste qui suit, on a rassemblé un certain nombre d'autres composés de formule (I) conformes à l'invention qui ont été préparés selon les modes opératoires décrits dans les exemples 1 et 2. Dans tous les cas, les résultats des analyses et les spectres de RMN ont confirmé la structure des composés.

### Exemple 4 :

N-[(N-éthyl-2-pyrrolidinyl)-méthyl]-1-méthoxy-2-naphtamide.
C₁₉H₂₆N₂O₃, F 85°C.

### Exemple 5 :

Dichlorhydrate de la 4-amino-N-[(N-éthyl-2-pyrrolidinyl)-méthyl]-1-méthoxy-2-naphtamide.
C₁₉H₂₅N₃O₂, 2HCl,H₂O, F 207°C.

### Exemple 6 :

4-Nitro-N-[(n-éthyl-2-pyrrolidinyl)-méthyl]-1-méthoxy-2-naphtamide.
C₁₉H₂₃N₃O₄, F 98°C.

### Exemple 7 :

Chlorhydrate du N-[(N-éthyl-2-pyrrolidinyl)-méthyl]-1,4-diméthoxy-2-naphtamide.
C₂₀H₂₇ClN₂O₃, F 158°C.

### Exemple 8 :

Chlorhydrate du 4-chloro-N-[(n-éthyl-2-pyrrolidinyl) -méthyl]-1-méthoxy-2-naphtamide.
C₁₉H₂₃BrN₂O₂,HCl,H₂O, F 188°C.

### Exemple 9 :

Chlorhydrate du 4-bromo-N-[(N-éthyl-2-pyrrolidinyl) -méthyl]-1-méthoxy-2-naphtamide.
C₁₉H₂₃BrN₂O₂,HCl,H₂O, F 194°C.

### Exemple 10 :

Chlorhydrate du 4-méthane sulfonylamino-N-[(N-éthyl-2-pyrrolidinyl)-méthyl]-1-méthoxy-2-naphtamide.
C₂₀H₂₇N₃O₄S,HCl, F 229°C

### Exemple 11 :

Chlorhydrate du N-[(N-benzyl-2-pyrrolidinyl)-méthyl]--1-méthoxy-2-naphtamide.
C₂₄H₂₆N₂O₂,HCl, F 145°C.

### Exemple 12 :

4-Nitro-N-[(N-benzyl-2-pyrrolidinyl)-méthyl]-1-méthoxy-2-naphtamide.
C₂₄H₂₅N₃O₄, F 123°C.

### Exemple 13 :

4-Chloro-N-[(N-benzyl-2-pyrrolidinyl)-méthyl]-1-méthoxy-2-naphtamide.
C₂₄H₂₅N₂O₂Cl, F 102°C.

### Exemple 14 :

4-Chloro-N-[(N-propyl-2-pyrrolidinyl)-méthyl]-1-méthoxy-2-naphtamide.
C₂₀H₂₅N₂O₂Cl, F 62°C.

### Exemple 15 :

4-Bromo-N-[(N-cyclopropylméthyl-2-pyrrolidinyl)-méthyl]-1-méthoxy-2-naphtamide.
C₂₁H₂₅N₂O₂Br, F 86°C.

### Exemple 16 :

4-Chloro-N-[(N-allyl-2-pyrrolidinyl)-méthyl]-1-méthoxy-2-naphtamide.
C₂₀H₂₃N₂O₂Cl, F 68°C.

### Exemple 17 :

4-Nitro-N-[(N-allyl-2-pyrrolidinyl)-méthyl]-1-méthoxy-2-naphtamide.
C₂₀H₂₃N₃O₄, F 90°C.

### Exemple 18 :

4-Cyano-N-[(Néthyl-2-pyrrolidinyl)-méthyl]-1-méthoxy-2-naphtamide.
C₂₀H₂₃N₃O₂, F 84°C.

### Exemple 19 :

4-Cyano-N[(N(3-butényl)-2-pyrrolidinyl)-méthyl-]-méthoxy-2-naphtamide
PF 115°C, C₂₂H₂₅N₃O₂

### Exemple 20 :

4-Cyano-N[(N-pentyl-2-pyrrolidinyl)-méthyl-]-méthoxy-2-naphtamide
PF 74°C, C₂₃H₂₉N₃O₂

### Exemple 21 :

4-Cyano-N[(N4-pentényl)-2-pyrrolidinyl)-méthyl-]-méthoxy-2-naphtamide
PF 66°C,C₂₃H₂₇N₃O₂

### Exemple 22 :

Chlorhydrate du 1,5-diméthoxy-N[(N-éthyl-2-pyrrolidinyl)-méthyl]2-naphtamide
PF 185°C, C₂₀H₂₇N₂ClO₃

### Exemple 23 :

Chlorhydrate du 1,5-diméthoxy-N[(N-butyl-2-pyrrolidinyl)-méthyl-2-naphtamide
PF 131°C,C₂₂H₃₁N₂ClO₃

### Exemple 24 :

4-Bromo-N[(N,N-diéthylamino)-éthyl]-1-méthoxy-2-naphtamide
PF 54°C,C₁₈H₂₃BrN₂O₂

### Exemple 25 :

4-Bromo-N[(morpholino)éthyl]-1-méthoxy-2-naphtamide
PF 87°C, C₁₈H₂₁N₂BrO₃

### Exemple 26 :

4-Bromo-N[(N-butyl)-4'-piperidinyl]-1-méthoxy-2-naphtamide
PF 121°C, C₂₁H₂₇N₂BrO₂

### Exemple 27 :

4-Bromo-N[(N-benzyl)-4'-piperidinyl]-1-méthoxy-2-naphtamide
PF 157°C, C₂₄H₂₅N₂OBr

### Exemple 28 :

4-Nitro-N[(morpholino)-éthyl]-1-méthoxy-2-naphtamide
PF 92°C, C₁₈H₂₁N₃O₅

### Exemple 29 :

Chlorhydrate du 4-nitro-N[(N-butyl-2-pyrrolidinyl)-méthyl]-1,5-diméthoxy-2-naphtamide
PF 102°C, C₂₂H₃₀N₃O₅Cl 1,5 H₂O

### Exemple 30 :

4-Nitro-N[N(butyl)-4'-piperidinyl]-1-méthoxy-2-naphtamide
PF 134°C, C₂₁H₂₇N₃O₄

### Exemple 31 :

4-Nitro-N[N,N-diéthylamino)-éthyl]-1-méthoxy-2-naphtamide
PF 84°C, C₁₈H₂₃N₃O₄

### Exemple 32 :

Chlorydrate de la 4-bromo-N[(N,N-dibutylamino)-éthyl]-1-méthoxy-2-naphtamide
PF 106°C, C₂₂H₃₂N₂O₂BrCl

### Exemple 33 :

4-Bromo-N(N-éthyl-2-pyrrolidinyl)-méthyl]-butoxy-2-naphtamide
PF 92°C, C₂₂H₂₉O₂N₂Br

### Exemple 34 :

4-Nitro-N[(N-éthyl-2-pyrrolidinyl)-méthyl]-1,5-dinitro-2-naphtamide
PF 57°C, C₂₀H₃₅N₃O₅

### Exemple 35 :

4-Bromo-N(N-butyl)-4'piperidinyl]-1-allyloxy-2-naphtamide
PF 72°C, C₂₃H₂₉N₂O₂Br

### Exemple 36 :

Chlorhydrate du 4-bromo-N[morpholinoéthyl)-1-allyloxy-2-naphtamide
PF 93°C, C₂₀H₂₄N₂O₃BrCl

### Exemple 37 :

Chlorhydrate du 1,4-diméthoxy-N[(N-butyl)-4'piperidinyl]-2-naphtamide
PF 137°C, C₂₂H₃₁N₂O₃Cl

### Exemple 38 :

Chlorhydrate du 4-bromo-N[(morpholinoéthyl)-1-butoxy]-2-naphtamide
PF 149°C, C₂₁H₂₈N₂O₃BrCl

### Exemple 39 :

Chlorhydrate du 4-bromo-N[(N-éthyl-2-pyrrolidinyl)-méthyl]-1-cyclopentyl-oxy-2-naphtamide
PF 138°C, C₂₃H₃₀N₂O₂BrCl

### Exemple 40 :

Chlorhydrate du 4-nitro-N[N,N-dibutylamino)éthyl)]-1-méthoxy-2-naphtamide
PF 113°C, C₂₂H₃₂N₃O₄Cl

### Exemple 41 :

4-Bromo-N(2-pyrrolidinyl éthyl)]-1-méthoxy-2-naphtamide
PF 91°C, C₁₈H₂₁N₂O₂Br

### Exemple 42 :

Chlorhydrate du 4-cyano-N[(N,N-dibutylamino)éthyl]-1-méthoxy-2-naphtamide
PF 86°C, C₂₃H₃₂N₃O₂Cl

Les composés de formule (I) conformes à l'invention ont été soumis à des tests pharmacologiques qui ont permis de mettre en évidence leurs propriétés antidopaminergiques.

### Mesure de l'affinité pour le récepteur D₃ de la dopamine.

Les constantes d'inhibition de la liaison de l'iodosulpiride [¹²⁵I] au récepteur D₃ ont été mesurées comme décrit dans la demande de brevet français déposée par l'INSERM le 6 novembre 1990 sous le n°90 13731. Les résultats obtenus ont été rassemblés dans le tableau qui suit. Il apparaît que les composés de l'invention ont une affinité élevée pour le récepteur D₃, qui pour certains d'entre eux, est meilleure que celle mesurée pour l'halopéridol et le sulpiride précédemment décrits.

### Activité anti-dopaminergique in vivo.

La capacité à exercer un blocage des récepteurs de la dopamine in vivo a été évaluée pour certains des composés conformes à l'invention. Les tests utilisés ont été l'antagonisme de la verticalisation et des reniflements stéréotypés induits par l'apomorphine chez la souris. Ces tests ont été décrits par Protais et coll. (Psychopharmacology, 1976, 50 : 1-6) et par Vasse et coll. (Naunyn Schmiedeberg's Arch. Pharmacol., 1985, 329 : 108-116). A titre d'exemple, les DI₅₀ du composé de l'exemple 8 pour l'antagonisme de la verticalisation et des reniflements sont respectivement de 0,16 mg/kg i.p. et de 0,42 mg/kg i.p., ce qui indique que le composé passe aisément la barrière hémato-encéphalique et exerce un blocage effectif des récepteurs de la dopamine.

Les résultats obtenus dans les tests pharmacologiques, qu'ils soient effectués in vivo ou in vitro, montrent que les composés de l'invention sont de nouveaux antagonistes de la dopamine actifs sur le récepteur D₃ de la dopamine. L'expression sélective de ce récepteur dans les régions limbiques du cerveau, susceptibles d'être affectées dans diverses maladies psychiatriques, ainsi que l'affinité élevée de la plupart des neuroleptiques ou antipsychotiques pour ce récepteur, suggèrent que le blocage du récepteur D₃ joue un rôle crucial dans le traitement de ces maladies.

La présente invention concerne donc également un médicament antagoniste de la dopamine par blocage du récepteur D₃ contenant, à titre de principe actif, l'un au moins des composés de formule générale (I), destiné à être utilisé comme antipsychotique, antidépresseur, psychostimulant, antiautistique, antiparkinsonien ou encore antihypertenseur.

L'invention concerne également l'utilisation de ces composés pour la préparation de ces médicaments, comprenant l'incorporation du composé dans un véhicule ou excipient oral ou parentéral, y compris la mise sous doses individuelles.

Les composés de formule (I) conformes à l'invention peuvent être administrés par voie orale ou parentérale, sous la forme de compositions pharmaceutiques contenant le principe actif en association avec un véhicule ou un excipient pharmaceutique acceptable. Les composés de l'invention sont actifs à des doses journalières comprises entre 0,01 et 10 mg/kg.

**TABLEAU**

| **CONSTANTES D'INHIBITION DE DIVERS COMPOSES DE L'INVENTION POUR LA LIAISON AU RECEPTEUR DOPAMINERGIQUE D**_{**3**} **HUMAIN, COMPAREES A CELLE DU HALOPERIDOL ET DU SULPIRIDE.** | |
|---|---|
| COMPOSE | VALEUR DE Kᵢ (nM) |
| Exemple n° 1 | 0,24 |
| n° 3 | 0,51 |
| n° 4 | 35 |
| n° 6 | 0,30 |
| n° 8 | 0,26 |
| n° 11 | 91 |
| n° 12 | 1,7 |
| n° 13 | 4,7 |
| n° 17 | 0,35 |
| Halopéridol | 2,9 |
| Sulpiride | 20 |

## Revendications

1. Médicament antagoniste de la dopamine par blocage du récepteur D₃, caractérisé en ce qu'il contient au moins un composé de formule (I) 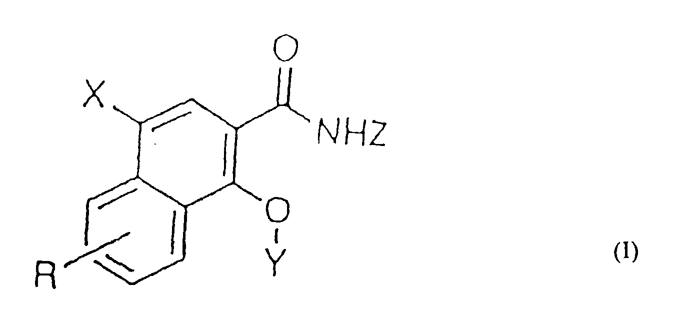 où
X représente soit un atome d'hydrogène, soit un atome de chlore ou de brome soit un groupement amino ou amino-alkyle, un groupe méthane sulfonylamino, un groupe thiocyanate, alkylthio, alkylsulfinyle ou alkylsulfonyle, soit un groupe méthoxy, soit un groupe nitro, soit un groupe cyano,
Y représente un reste alkyle ou alcényle
Z représente les restes 2-méthyl N-(alkyl en C₁-C₆) pyrrolidine, 2-éthyl-N,N-diéthylamine, 2-éthyl morpholine, 2-éthyl-N,N-dibutylamine, 4-N-butyl pipéridine, 4-N-benzyl pipéridine, 2-éthylpyrrolidine.
R est un hydrogène ou un substituant OCH₃,
sous réserve que lorsque Z est un reste 2-éthyl-N,N-diéthylamine et R est un hydrogène, X est différent du groupe méthoxy et Y est différent du groupe methyle.

2. Médicament selon la revendication 1, caractérisé en ce qu'il contient au moins un composé de formule (Ia) 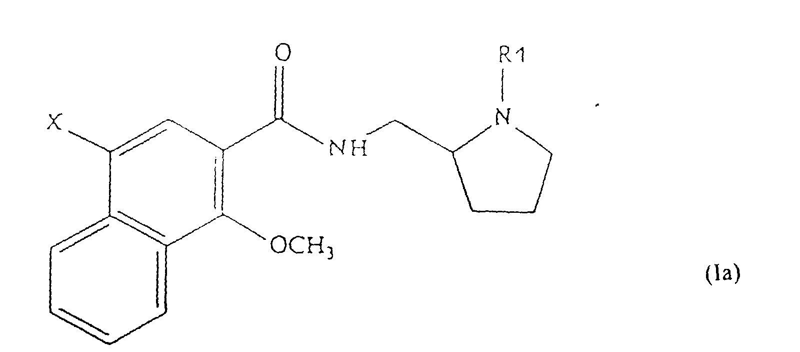 où
X représente un atome d'hydrogène, un atome de chlore ou de brome, un groupe amino ou amino-alkyle, un groupe méthane sulfonylamino, un groupe méthoxy, un groupe nitro ou encore un groupe cyano,
R1 représente un groupe alkyle en C₁-C₆,
ainsi que leurs sels d'addition avec les acides physiologiquement acceptables.

3. Médicament selon la revendication 2, caractérisé en ce que le composé de formule (Ia) est tel que X représente un atome d'hydrogène, un atome de chlore, un groupe amino, nitro, cyano ou encore un groupe méthoxy et R1 représente un radical éthyle, propyle ou butyle.

4. Médicament selon l'une des revendications 1 à 3, caractérisé en ce que le composé (I) est choisi dans le groupe constitué par :
- le N-[(N-1-butyl-2-pyrrolidinyl)-méthyl]-1-méthoxy-4-bromo-2-naphtamide, éventuellement sous forme de chlorhydrate,
- le N-[(N-éthyl-2-pyrrolidinyl)-métyl]-1-méthoxy -2-naphtamide,
- le 4-amino-N-[(N-éthyl-2-pyrrolydinyl)-méthyl]-1-méthoxy-2-naphtamide, éventuellement sous forme de dichlorhydrate,
- le 4-nitro-N-[(N-éthyl-2-pyrrolydinyl)-méthyl]-1-méthoxy-2-naphtamide,
- le N-[(N-éthyl-2-pyrrolidinyl)-méthyl]-1,4-diméthoxy-2-naphtamide, éventuellement sous forme de chlorhydrate,
- le 4-chloro-N-[(N-éthyl-2-pyrrolidinyl)-méthyl] -1-méthoxy-2-naphtamide, éventuellement sous forme de chlorhydrate,
- le 4-bromo-N-[(N-éthyl-2-pyrrolidinyl)-méthyl] -1-méthoxy-2-naphtamide, éventuellement sous forme de chlorhydrate,
- le 4-méthane sulfonylamino-N-[(N-éthyl-2-pyrrolidinyl)-méthyl]-1-méthoxy-2-naphtamide, éventuellement sous forme de chlorhydrate,
- le 4-chloro-N-[(N-propyl-2-pyrrolidinyl)-méthyl] -1-méthoxy-2-naphtamide,
- le 4-cyano-N-[(N-éthyl-2-pyrrolidinyl)-méthyl] -1-méthoxy-2-naphtamide.
- le 4-cyano-N-[(N-butyl-2-pyrrolidinyl)-méthyl]-1-méthoxy-2-naphtamide
- le 4-cyano-N-[(N-pentyl-2-pyrrolidinyl)-méthyl]-1-méthoxy-2-naphtamide
- le Chlorhydrate du 1,5-diméthoxy-N[(N-éthyl-2-pyrrolidiny)-méthyl]-2-naphtamide
- le Chlorhydrate du 1,5-diméthoxy-N[(N-butyl-2-pyrrolidiny)-méthyl]-2-naphtamide
- le 4-Bromo-N[(N,N-diéthylamino)-éthyl]-1-méthoxy-2-naphtamide
- le 4-Bromo-N[(morpholino)éthyl]-1-méthoxy-2-naphtamide
- le 4-Bromo-N[(N-butyl)-4'-piperidinyl]-1-méthoxy-2-naphtamide
- le 4-Bromo-N[(N-benzyl)-4'piperidinyl]-1-methoxy-2 naphtamide
- le 4-Nitro-N[(morpholino)-éthyl]-1-méthoxy-2-naphtamide
- le Chlorhydrate du 4-nitro-N[(N-butyl-2-pyrrolidinyl -méthyl]-1,5-diméthoxy-2-naphtamide
- le 4-Nitro-N[N(butyl)-4'piperidinyl]-1-méthoxy-2-naphtamide
- le 4-Nitro-N[N,N-diéthylamino)-éthyl]-1-méthoxy-2-naphtamide
- le Chlorhydrate de la 4-bromo-N[N,N-dibutylamino)-éthyl]-1-méthoxy-2-naphtamide
- le 4-Bromo-N[(N-éthyl-2-pyrrolidinyl)-méthyl]-1-butoxy-2-naphtamide
- le 4-Nitro-N[(N-éthyl-2-pyrrolidinyl)-méthyl]-115-dinitro-2-naphtamide
- le 4-Bromo-N[(N-butyl)-4'piperidinyl]-1-allyloxy-2-naphtamide
- le Chlorhydrate du 4-bromo-N[(morpholinoéthyl)-1-allyloxy-2-naphtamide
- le Chlorhydrate du 1,4 diméthoxy-N[(N-butyl)-4'piperidinyl]-2-naphtamide
- le Chlorhydrate du 4-bromo-N[(morpholino)éthyl)-1-butoxy]-2-naphtamide
- le Chlorhydrate du 4-bromo-N[(N-éthyl-2-pyrrolidinyl) -méthyl]-1-cyclopentyl-oxy-2-naphtamide
- le Chlorhydrate du 4-nitro-N[(N,N-dibutylamino)éthyl] -1-méthoxy-2-naphtamide
- le 4-Bromo-N[(2-pyrrolidinyl éthyl)]-1-méthoxy-2-naphtamide
- le Chlorhydrate du 4-cyano-N[(N,N-dibutylamino)éthyl] -1-méthoxy-2-naphtamide
- le chlorhydrate du 4-cyano-N-[(N,N-dibutylamino) éthyl]-1-méthoxy-2-naphtamide

5. Utilisation d'un dérivé de formule (I) 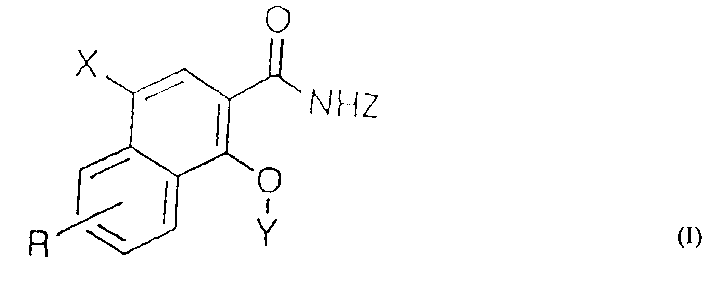 où
X représente soit un atome d'hydrogène, soit un atome de chlore ou de brome, soit un groupement amino ou amino-alkyle, un groupe méthane sulfonylamino, un groupe thiocyanate, alkylthio, alkylsulfinyle ou alkylsulfonyle, soit un groupe méthoxy, soit un groupe nitro, soit un groupe cyano,
Y représente un reste alkyle ou alcényle
Z représente les restes 2-méthyl N-(alkyl en C₁-C₆,) pyrrolidine, 2-éthyl-N,N-diéthylamine, 2-éthyl morpholine, 2-éthyl-N,N-dibutylamine, 4-N-butyl pipéridine, 4-N-benzyl pipéridine, 2-éthylpyrrolidine.
R est un hydrogène ou un substituant OCH₃.
pour la préparation d'un médicament destiné à être utilisé comme agent antipsychotique, psychostimulant, antiautistique, anti-dépresseur, antiparkinsonien ou antihypertenseur.

6. Utilisation selon la revendication 5, caractérisée en ce que le dérivé (1) répond à la formule (Ia) 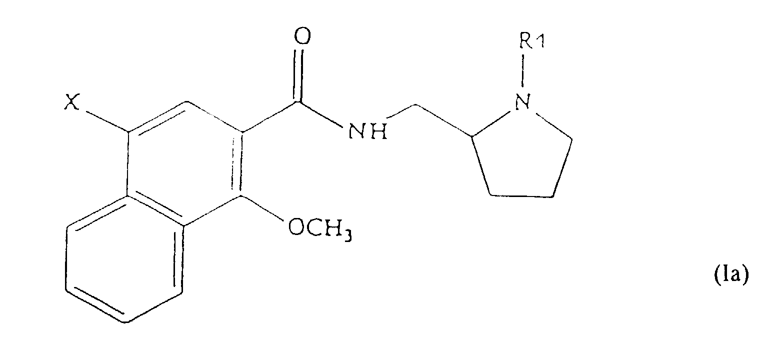 où
X représente un atome d'hydrogène, un atome de chlore ou de brome un groupe amino ou amino-alkyle, un groupe méthane sulfonylamino, un groupe méthoxy, un groupe nitro ou encore un groupe cyano,
R1 représente un groupe alkyle en C₁-C₆,
ainsi que leurs sels d'addition avec les acides physiologiquement acceptables.

7. Utilisation selon la revendication 6, caractérisée en ce que le composé de formule (la) est tel que X représente un atome d'hydrogène, un atome de chlore, un groupe amino, nitro, cyano ou encore un groupe méthoxy et R1 représente un radical éthyle, propyle ou butyle.

8. Utilisation selon l'une quelconque des revendications 5 à 7, caractérisée en ce que le dérivé de formule (I) est choisi dans le groupe constitué par:
- le N-[(N-1-butyl-2-pyrrolidinyl)-méthyl]-1-méthoxy-4-bromo-2-naphtamide, éventuellement sous forme de chlorhydrate,
- le N-[(N-éthyl-2-pyrrolidinyl)-méthyl]-1-méthoxy -2-naphtamide,
- le 4-amino-N-[(N-éthyl-2-pyrrolydinyl)-méthyl]-1-méthoxy-2-naphtamide, éventuellement sous forme de dichlorhydrate,
- le 4-nitro-N-[(N-éthyl-2-pyrrolydinyl)-méthyl]-1-méthoxy-2-naphtamide,
- le N-[(N-éthyl-2-pyrrolidinyl)-méthyl]-1,4-diméthoxy-2-naphtamide, éventuellement sous forme de chlorhydrate,
- le 4-chloro-N-[(N-éthyl-2-pyrrolidinyl)-méthyl] -1-méthoxy-2-naphtamide, éventuellement sous forme de chlorhydrate,
- le 4-bromo-N-[(N-éthyl-2-pyrrolidinyl)-méthyl] -1-méthoxy-2-naphtamide, éventuellement sous forme de chlorhydrate,
- le 4-méthane sulfonylamino-N-[(N-éthyl-2-pyrrolidinyl)-méthyl]-1-méthoxy-2-naphtamide, éventuellement sous forme de chlorhydrate,
- le 4-chloro-N-[(N-propyl-2-pyrrolidinyl)-méthyl] -1-méthoxy-2-naphtamide,
- le 4-cyano-N-[(N-éthyl-2-pyrrolidinyl)-méthyl] -1-méthoxy-2-naphtamide.
- le 4-cyano-N-[(N-butyl-2-pyrrolidinyl)-méthyl]-1-méthoxy-2-naphtamide
- le 4-cyano-N-[(N-pentyl-2-pyrrolidinyl)-méthyl]-1-méthoxy-2-naphtamide
- le Chlorhydrate du 1,5-diméthoxy-N[(N-éthyl-2-pyrrolidiny)-méthyl]-2-naphtamide
- le Chlorhydrate du 1,5-diméthoxy-N[(N-butyl-2-pyrrolidiny)-méthyl]-2-naphtamide
- le 4-Bromo-N[(N,N-diéthylamino)-éthyl]-1-méthoxy-2-naphtamide
- le 4-Bromo-N[(morpholino)éthyl]-1-méthoxy-2-naphtamide
- le 4-Bromo-N[(N-butyl)-4'-piperidinyl]-1-méthoxy-2-naphtamide
- le 4-Bromo-N[(N-benzyl)-4'piperidinyl]-1-methoxy-2 naphtamide
- le 4-Nitro-N[(morpholino)-éthyl]-1-méthoxy-2-naphtamide
- le Chlorhydrate du 4-nitro-N[(N-butyl-2-pyrrolidinyl) -méthyl]-1,5-diméthoxy-2-naphtamide
- le 4-Nitro-N[N(butyl)-4'piperidinyl]-1-méthoxy-2-naphtamide
- le 4-Nitro-N[N,N-diéthylamino)-éthyl]-1-méthoxy-2-naphtamide
- le Chlorhydrate de la 4-bromo-N[N,N-dibutylamino)-éthyl]-1-méthoxy-2-naphtamide
- le 4-Bromo-N[(N-éthyl-2-pyrrolidinyl)-méthyl]-1-butoxy-2-naphtamide
- le 4-Nitro-N[(N-éthyl-2-pyrrolidinyl)-méthyl]-1,5-dinitro-2-naphtamide
- le 4-Bromo-N[(N-butyl)-4'piperidinyl]-1-allyloxy-2-naphtamide
- le Chlorhydrate du 4-bromo-N[(morpholinoéthyl)-1-allyloxy-2-naphtamide
- le Chlorhydrate du 1,4 diméthoxy-N[(N-butyl)-4'piperidinyl]-2-naphtamide
- le Chlorhydrate du 4-bromo-N[(morpholino)éthyl)-1-butoxy]-2-naphtamide
- le Chlorhydrate du 4-bromo-N[(N-éthyl-2-pyrrolidinyl) -méthyl]-1-cyclopentyl-oxy-2-naphtamide
- le Chlorhydrate du 4-nitro-N[(N,N-dibutylamino)éthyl] -1-méthoxy-2-naphtamide
- le 4-Bromo-N[(2-pyrrolidinyl éthyl)]-1-méthoxy-2-naphtamide
- le Chlorhydrate du 4-cyano-N[(N,N-dibutylamino)éthyl] -1-méthoxy-2-naphtamide
- le chlorhydrate du 4-cyano-N-[(N,N-dibutylamino) éthyl]-1-méthoxy-2-naphtamide

## Patentansprüche

1. Durch Blockierung des D₃-Rezeptors gegenüber Dopamin antagonistisches Medikament, dadurch gekennzeichnet, daß es mindestens eine Verbindung der Formel (I) enthält, 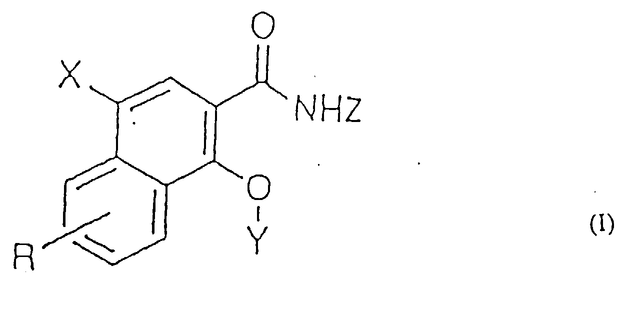 in der
X entweder ein Wasserstoffatom oder ein Chlor- oder Bromatom oder eine Amino- oder Aminoalkylgruppe, eine Methansulfonylaminogruppe, eine Thiocyanat-, Alkylthio-, Alkylsufinyl- oder Alkylsulfonylgruppe oder eine Methoxygruppe oder eine Nitrogruppe oder eine Cyanogruppe bedeutet,
Y einen Alkyl- oder Alkenylrest bedeutet,
Z für die Reste 2-Methyl-N-(alkyl[C₁-C₆])-pyrrolidin, 2-Ethyl-N,N-diethylamin, 2-Ethyl-morpholin, 2-Ethyl-N,N-dibutylamin, 4-N-Butylpiperidin, 4-N-Benzylpiperidin und 2-Ethylpyrrolidin steht,
R ein Wasserstoffatom oder ein OCH₃-Substituent ist,
unter dem Vorbehalt, daß, wenn Z ein 2-Ethyl-N,N-diethylamin-Rest und R ein Wasserstoffatom ist, X keine Methoxygruppe und Y keine Methylgruppe ist.

2. Arzneimittel nach Anspruch 1, dadurch gekennzeichnet, daß es mindestens eine Verbindung der Formel (Ia) enthält 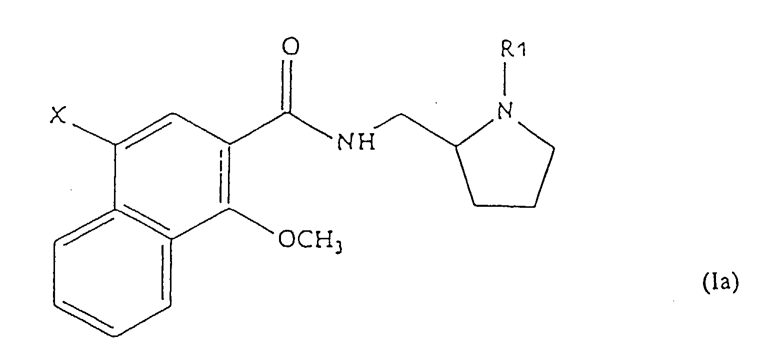 in der X ein Wasserstoffatom, ein Chlor- oder Bromatom, eine Amino- oder Aminoalkylgruppe, eine Methansulfonylamino-Gruppe, eine Methoxygruppe, eine Nitrogruppe oder auch eine Cyanogruppe bedeutet, R₁ für eine C₁-C₆-Alkylgruppe steht, sowie ihre Additionssalze mit physiologisch unbedenklichen Säuren.

3. Arzneimittel nach Anspruch 2, dadurch gekennzeichnet, daß die Verbindung gemäß Formel (Ia) so vorliegt, daß X ein Wasserstoffatom, ein Chloratom, eine Amino-, Nitro-, Cyano- oder auch eine Methoxygruppe ist und R₁ für ein Ethyl-, Propyl- oder Butylradikal steht.

4. Arzneimittel nach einem der Ansprüche 1 bis 3, dadurch gekennzeichnet, daß die Verbindung (I) ausgewählt wird aus der Gruppe, bestehend aus:
- N-[(N-1-Butyl-2-pyrrolidinyl)-methyl]-1-methoxy-4-brom-2-naphthamid, eventuell in Form des Chlorhydrats;
- N-[(N-Ethyl-2-pyrrolidinyl)-methyl]-1-methoxy-2-naphthamid;
- 4-Amino-N-[(N-ethyl-2-pyrrolidinyl)-methyl]-1-methoxy-2-naphthamid, eventuell in Form des Dichlorhydrats; 4-Nitro-N-[(N-ethyl-2-pyrrolidinyl)-methyl]-1-methoxy-2-naphthamid;
- N-[(N-Ethyl-2-pyrrolidinyl)-methyl]-1,4-dimethoxy-2-naphthamid, eventuell in Form des Chlorhydrats;
- 4-Chlor-N-[(N-ethyl-2-pyrrolidinyl)-methyl]-1-methoxy-2-naphthamid, eventuell in Form des Chlorhydrats;
- 4-Brom-N-[(N-ethyl-2-pyrrolidinyl-methyl]-1-methoxy-2-naphthamid, eventuell in Form des Chlorhydrats;
- 4-Methansulfonylamino-N-[(N-ethyl-2-pyrrolidinyl)-methyl]-1-methoxy-2-naphthamid, eventuell in Form des Chlorhydrats;
- 4-Chlor-N-[(N-propyl-2-pyrrolidinyl)-methyl]-1-methoxy-2-naphthamid;
- 4-Cyano-N-[(N-ethyl-2-pyrrolidinyl)-methyl]-1-methoxy-2-naphthamid;
- 4-Cyano-N-[(N-butyl-2-pyrrolidinyl)-methyl]-1-methoxy-2-naphthamid;
- 4-Cyano-N-[(N-pentyl-2-pyrrolidinyl)-methyl]-1-methoxy-2-naphthamid;
- Chlorhydrat von 1,5-Dimethoxy-N-[(N-ethyl-2-pyrrolidinyl)-methyl]-2-naphthamid;
- Chlorhydrat von 1,5-Dimethoxy-N-[(N-butyl-2-pyrrolidinyl)-methyl]-2-naphthamid;
- 4-Brom-N-[(N,N-diethylamino)-ethyl]-1-methoxy-2-naphthamid;
- 4-Brom-N-[(morpholino)-ethyl]-1-methoxy-2-naphthamid;
- 4-Brom-N-[(N-butyl)-4'-piperidinyl]-1-methoxy-2-naphthamid;
- 4-Brom-N-[(N-benzyl)-4'-piperidinyl]-1-methoxy-2-naphthamid;
- 4-Nitro-N-[(morpholino)-ethyl]-1-methoxy-2-naphthamid;
- Chlorhydrat von 4-Nitro-N-[(N-butyl-2-pyrrolidinyl)-methyl]-1,5-dimethoxy-2-naphthamid;
- 4-Nitro-N-[(N-butyl)-4'-piperidinyl]-1-methoxy-2-naphthamid;
- 4-Nitro-N-[(N,N-diethylamino)-ethyl]-1-methoxy-2-naphthamid;
- Chlorhydrat von 4-Brom-N-[(N,N-dibutylamino)-ethyl]-1-methoxy-2-naphthamid;
- 4-Brom-N-[(N-ethyl-2-pyrrolidinyl)-methyl]-1-butoxy-2-naphthamid;
- 4-Nitro-N-[(N-ethyl-2-pyrrolidinyl)-methyl]-1,5-dinitro-2-naphthamid;
- 4-Brom-N-[(N-butyl)-4'-piperidinyl]-1-allyloxy-2-naphthamid;
- Chlorhydrat von 4-Brom-N-[(morpholino)-ethyl]-1-allyloxy-2-naphthamid;
- Chlorhydrat von 1,4-Dimethoxy-N-[(N-butyl)-4'-piperidinyl]-2-naphthamid;
- Chlorhydrat von 4-Brom-N-[(morpholino)-ethyl]-1-butoxy-2-naphthamid;
- Chlorhydrat von 4-Brom-N-[(N-ethyl-2-pyrrolidinyl)-methyl]-1-cyclopentyloxy-2-naphthamid;
- Chlorhydrat von 4-Nitro-N-[(N,N-dibutylamino)-ethyl]-1-methoxy-2-naphthamid;
- 4-Brom-N-[(2-pyrrolidinyl)-ethyl]-1-methoxy-2-naphthamid;
- Chlorhydrat von 4-Cyano-N-[(N,N-dibutylamino)-ethyl]-1-methoxy-2-naphthamid.

5. Verwendung eines Derivats der Formel (I), 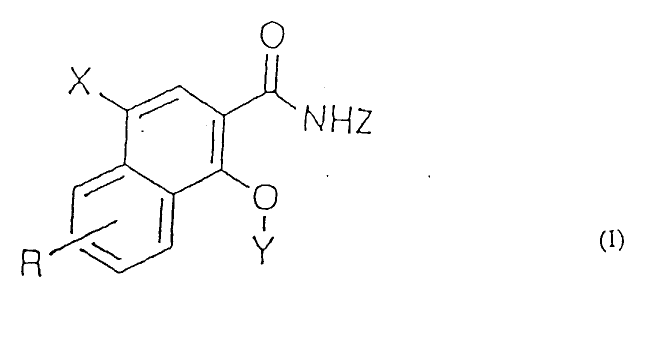 in dem
X entweder ein Wasserstoffatom, oder ein Chlor- oder Bromatom, oder eine Amino- oder Aminoalkylgruppe, eine Methansulfonylaminogruppe, eine Thiocyanat-, Alkylthio-, Alkylsulfinyl- oder Alkylsulfonylgruppe oder eine Methoxygruppe oder eine Nitrogruppe oder eine Cyanogruppe bedeutet,
Y einen Alkyl- oder Alkenylrest bedeutet,
Z für die Reste 2-Methyl-N-(C₁-C₆-alkyl)-pyrrolidin, 2-Ethyl-N,N-diethylamin, 2-Ethylmorpholin, 2-Ethyl-N,N-dibutylamin, 4-N-Butylpiperidin, 4-N-Benzylpiperidin und 2-Ethylpyrrolidin steht,
R ein Wasserstoffatom oder ein OCH₃-Substituent ist, für die Herstellung eines Arzneimittels zur Verwendung als antipsychotisches, psychostimulierendes,
antiautistisches, antidepressives, gegen Parkinson wirksames oder antihypertonisches Mittel.

6. Verwendung nach Anspruch 5, dadurch gekennzeichnet, daß das Derivat (I) der Formel (Ia) entspricht, 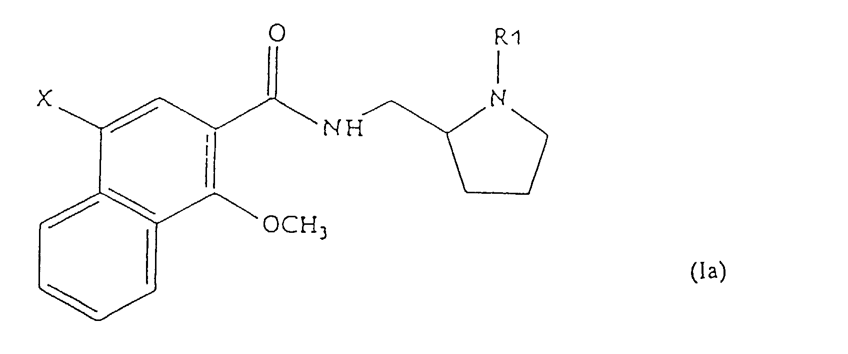 in der
X ein Wasserstoffatom, ein Chlor- oder Bromatom, eine Amino- oder Aminoalkylgruppe, eine Methansulfonylamino-gruppe, eine Methoxygruppe, eine Nitrogruppe oder auch eine Cyanogruppe bedeutet,
R₁ für eine C₁-C₆-Alkylgruppe steht, sowie ihre Additionssalze mit physiologisch unbedenklichen Säuren.

7. Verwendung nach Anspruch 6, dadurch gekennzeichnet, daß die Verbindung der Formel (Ia) so vorliegt, daß X ein Wasserstoffatom, ein Chloratom, eine Amino-, Nitro-, Cyano- oder auch eine Methoxygruppe ist, und R₁ für ein Ethyl-, Propyl- oder Butylradikal steht.

8. Verwendung nach einem der Ansprüche 5 bis 7, dadurch gekennzeichnet, daß das Derivat der Formel (I) ausgewählt wird aus der Gruppe, bestehend aus:
- N-[(N-1-Butyl-2-pyrrolidinyl)-methyl]-1-methoxy-4-brom-2-naphthamid, eventuell in Form des Chlorhydrats;
- N-[(N-Ethyl-2-pyrrolidinyl)-methyl]-1-methoxy-2-naphthamid;
- 4-Amino-N-[(N-ethyl-2-pyrrolidinyl)-methyl]-1-methoxy-2-naphthamid, eventuell in Form des Dichlorhydrats;
4-Nitro-N-[(N-ethyl-2-pyrrolidinyl)-methyl]-1-methoxy-2-naphthamid;
- N-[(N-Ethyl-2-pyrrolidinyl)-methyl]-1,4-dimethoxy-2-naphthamid, eventuell in Form des Chlorhydrats;
- 4-Chlor-N-[(N-ethyl-2-pyrrolidinyl)-methyl]-1-methoxy-2-naphthamid, eventuell in Form des Chlorhydrats;
- 4-Brom-N-[(N-ethyl-2-pyrrolidinyl)-methyl]-1-methoxy-2-naphthamid, eventuell in Form des Chlorhydrats;
- 4-Methansulfonylamino-N-[(N-ethyl-2-pyrrolidinyl)-methyl]-1-methoxy-2-naphthamid, eventuell in Form des Chlorhydrats;
- 4-Chlor-N-[(N-propyl-2-pyrrolidinyl)-methyl]-1-methoxy-2-naphthamid;
- 4-Cyano-N-[(N-ethyl-2-pyrrolidinyl)-methyl]-1-methoxy-2-naphthamid;
- 4-Cyano-N-[(N-butyl-2-pyrrolidinyl)-methyl]-1-methoxy-2-naphthamid;
- 4-Cyano-N-[(N-pentyl-2-pyrrolidinyl)-methyl]-1-methoxy-2-naphthamid;
- Chlorhydrat von 1,5-Dimethoxy-N-[(N-ethyl-2-pyrrolidinyl)-methyl]-2-naphthamid;
- Chlorhydrat von 1,5-Dimethoxy-N-[(N-butyl-2-pyrrolidinyl)-methyl]-2-naphthamid;
- 4-Brom-N-[(N,N-diethylamino)-ethyl]-1-methoxy-2-naphthamid;
- 4-Brom-N-[(morpholino)-ethyl]-1-methoxy-2-naphthamid;
- 4-Brom-N-[(N-butyl)-4'-piperidinyl]-1-methoxy-2-naphthamid;
- 4-Brom-N-[(N-benzyl)-4'-piperidinyl]-1-methoxy-2-naphthamid;
- 4-Nitro-N-[(morpholino)-ethyl]-1-methoxy-2-naphthamid;
- Chlorhydrat von 4-Nitro-N-[(N-butyl-2-pyrrolidinyl)-methyl]-1,5-dimethoxy-2-naphthamid;
- 4-Nitro-N-[(N-butyl)-4'-piperidinyl]-1-methoxy-2-naphthamid;
- 4-Nitro-N-[(N,N-diethylamino)-ethyl]-1-methoxy-2-naphthamid;
- Chlorhydrat von 4-Brom-N-[(N,N-dibutylamino)-ethyl]-1-methoxy-2-naphthamid;
- 4-Brom-N-[(N-ethyl-2-pyrrolidinyl)-methyl]-1-butoxy-2-naphthamid;
- 4-Nitro-N-[(N-ethyl-2-pyrrolidinyl)-methyl]-1,5-dinitro-2-naphthamid;
- 4-Brom-N-[(N-butyl)-4'-piperidinyl]-1-allyloxy-2-naphthamid;
- Chlorhydrat von 4-Brom-N-[(morpholino)-ethyl]-1-allyloxy-2-naphthamid;
- Chlorhydrat von 1,4-Dimethoxy-N-[(N-butyl)-4'-piperidinyl]-2-naphthamid;
- Chlorhydrat von 4-Brom-N-[(morpholino)-ethyl]-1-butoxy-2-naphthamid;
- Chlorhydrat von 4-Brom-N-[(N-ethyl-2-pyrrolidinyl)-methyl]-1-cyclopentyloxy-2-naphthamid;
- Chlorhydrat von 4-Nitro-N-[(N,N-dibutylamino)-ethyl]-1-methoxy-2-naphthamid;
- 4-Brom-N-[(2-pyrrolidinyl)-ethyl]-1-methoxy-2-naphthamid;
- Chlorhydrat von 4-Cyano-N-[(N,N-dibutylamino)-ethyl]-1-methoxy-2-naphthamid.

## Claims

1. Dopamine antagonist medication acting by blocking the D₃ receptor, characterised in that it contains at least one compound with the formula (1) 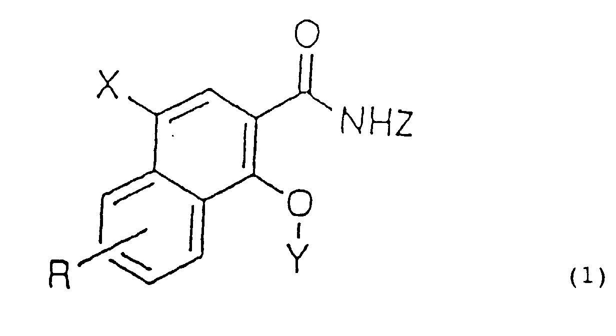 where
X represents either a hydrogen atom or a chlorine or bromine atom, or an amino or amino alkyl group, a methane sulphonyl amino group, a thiocyanate, alkylthio, alkylsulphinyl or alkylsulphonyl group, or a methoxy group, or a nitro group, or a cyano group,
Y represents an alkyl or alkenyl residue,
Z represents the residues 2-methyl N-(C₁-C₆ alkyl) pyrrolidine, 2-ethyl-N,N-diethylamine, 2-ethyl morpholine, 2-ethyl-N,N-dibutylamine, 4-N-butyl piperidine, 4-N-benzyl piperidine, 2-ethyl pyrrolidine,
R is a hydrogen or an OCH₃ substituent,
subject to Z being a 2-ethyl-N,N-diethylamine residue and R being a hydrogen, X being different from the methoxy group and Y being different from the methyl group.

2. Medication according to Claim 1, characterised in that it contains at least one compound with the formula (1a) 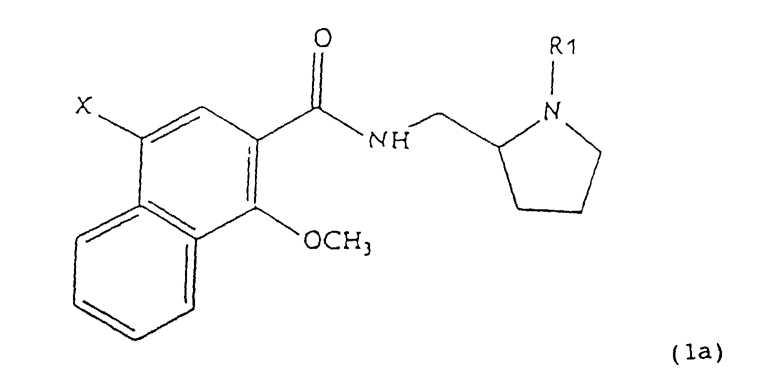 where
X represents a hydrogen atom, a chlorine or bromine atom, an amino or amino alkyl group, a methane sulphonyl amino group, a methoxy group, a nitro group, or also a cyano group,
R1 represents a C₁-C₆ alkyl group,
as well as their addition salts with physiologically acceptable acids.

3. Medication according to Claim 2, characterised in that the compound with the formula (1a) is such that X represents a hydrogen atom, a chlorine atom, an amino, nitro, cyano group or also a methoxy group, and R1 represents an ethyl, propyl or butyl radical.

4. Medication according to one of Claims 1 to 3, characterised in that the compound (1) is selected from the group comprising
- N-[(N-1-butyl-2-pyrrolidinyl)-methyl]-1-methoxy-4-bromo-2-naphthamide, possibly in the form of chlorohydrate,
- N-[(N-ethyl-2-pyrrolidinyl)-methyl]-1-methoxy-2-naphthamide,
- 4-amino-N-[(N-ethyl-2-pyrrolidinyl)-methyl]-1-methoxy-2-naphthamide, possibly in the form of dichlorohydrate,
- 4-nitro-N-[(N-ethyl-2-pyrrolidinyl)-methyl]-1-methoxy-2-naphthamide,
- N-[(N-ethyl-2-pyrrolidinyl)-methyl]-1,4-dimethoxy-2-naphthamide, possibly in the form of chlorohydrate,
- 4-chloro-N-[(N-ethyl-2-pyrrolidinyl)-methyl]-1-methoxy-2-naphthamide, possibly in the form of chlorohydrate,
- 4-bromo-N-[(N-ethyl-2-pyrrolidinyl)-methyl]-1-methoxy-2-naphthamide, possibly in the form of chlorohydrate,
- 4-methane sulphonyl amino-N-[(N-ethyl-2-pyrrolidinyl)-methyl]-1-methoxy-2-naphthamide, possibly in the form of chlorohydrate,
- 4-chloro-N-[(N-propyl-2-pyrrolidinyl)-methyl]-1-methoxy-2-naphthamide,
- 4-cyano-N-[(N-ethyl-2-pyrrolidinyl)-methyl]-1-methoxy-2-naphthamide,
- 4-cyano-N-[(N-butyl-2-pyrrolidinyl)-methyl]-1-methoxy-2-naphthamide,
- 4-cyano-N-[(N-pentyl-2-pyrrolidinyl)-methyl]-1-methoxy-2-naphthamide,
- 1,5-dimethoxy-N-[(N-ethyl-2-pyrrolidinyl)-methyl]-2-naphthamide chlorohydrate,
- 1,5-dimethoxy-N-[(N-butyl-2-pyrrolidinyl)-methyl]-2-naphthamide chlorohydrate,
- 4-bromo-N[(N,N-diethyl amino)-ethyl]-1-methoxy-2-naphthamide,
- 4-bromo-N[(morpholino)ethyl]-1-methoxy-2-naphthamide,
- 4-bromo-N[(N-butyl)-4'-piperidinyl]-1-methoxy-2-naphthamide,
- 4-bromo-N[(N-benzyl)-4'-piperidinyl]-1-methoxy-2-naphthamide,
- 4-nitro-N[(morpholino)ethyl]-1-methoxy-2-naphthamide,
- 4-nitro-N[(N-butyl-2-pyrrolidinyl)-methyl]-1,5-dimethoxy-2-naphthamide chlorohydrate,
- 4-nitro-N[(N-butyl)-4'piperidinyl]-1-methoxy-2-naphthamide,
- 4-nitro-N[(N,N-diethyl amino)-ethyl]-1-methoxy-2-naphthamide,
- 4-bromo-N[(N,N-dibutyl amino)-ethyl]-1-methoxy-2-naphthamide chlorohydrate,
- 4-bromo-N[(N-ethyl-2-pyrrolidinyl)-methyl]-1-butoxy-2-naphthamide,
- 4-nitro-N[(N-ethyl-2-pyrrolidinyl)-methyl]-1,5-dinitro-2-naphthamide,
- 4-bromo-N[(N-butyl)-4'piperidinyl]-1-allyloxy-2-naphthamide,
- 4-bromo-N[(morpholino)ethyl]-1-allyloxy-2-naphthamide chlorohydrate,
- 1,4 dimethoxy-N[(N-butyl)-4'piperidinyl]-2-naphthamide chlorohydrate,
- 4-bromo-N[(morpholino)ethyl]-1-butoxy-2-naphthamide chlorohydrate,
- 4-bromo-N[(N-ethyl-2-pyrrolidinyl)-methyl]-1-cyclopentyl-oxy-2-naphthamide chlorohydrate,
- 4-nitro-N[(N,N-dibutyl amino)-ethyl]-1-methoxy-2-naphthamide chlorohydrate,
- 4-bromo-N[(2-pyrrolidinyl)-ethyl]-1-methoxy-2-naphthamide,
- 4-cyano-N[(N,N-dibutyl amino)-ethyl]-1-methoxy-2-naphthamide chlorohydrate,
- 4-cyano-N[(N,N-dibutyl amino)-ethyl]-1-methoxy-2-naphthamide chlorohydrate.

5. Use of a derivative of formula (1) 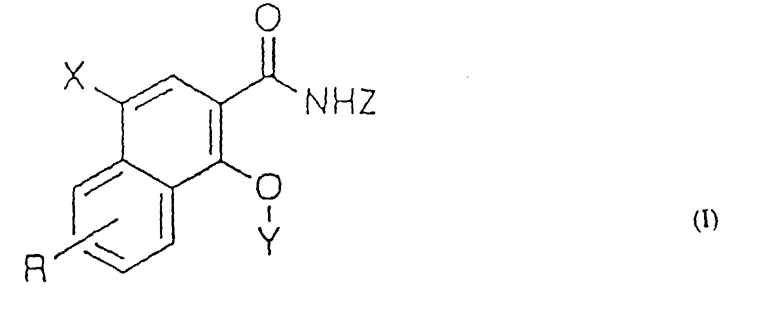 where
X represents either a hydrogen atom or a chlorine or bromine atom, or an amino or amino alkyl group, a methane sulphonyl amino group, a thiocyanate, alkylthio, alkylsulphinyl or alkylsulphonyl group, or a methoxy group, or a nitro group, or a cyano group,
Y represents an alkyl or alkenyl residue,
Z represents the residues 2-methyl N-(C₁-C₆ alkyl) pyrrolidine, 2-ethyl-N,N-diethylamine, 2-ethyl morpholine, 2-ethyl-N,N-dibutylamine, 4-N-butyl piperidine, 4-N-benzyl piperidine, 2-ethyl pyrrolidine,
R is a hydrogen or an OCH₃ substituent,
for the preparation of a medication intended for use as antipsychotic, psycho-stimulant, antiautistic, antidepressant, anti-parkinson or anti-hypertensive agent.

6. Use according to Claim 5, characterised in that the derivative (1) meets the formula (la) 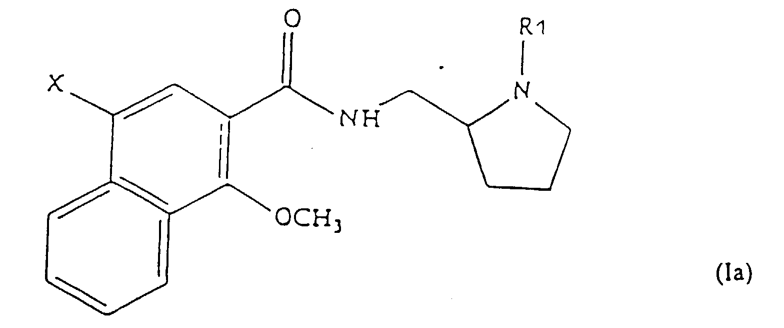 where
X represents a hydrogen atom, a chlorine or bromine atom, an amino or amino alkyl group, a methane sulphonyl amino group, a methoxy group, a nitro group, or also a cyano group,
R1 represents a C₁-C₆ alkyl group,
as well as their addition salts with physiologically acceptable acids.

7. Use according to Claim 6, characterised in that the compound with the formula (la) is such that X represents a hydrogen atom, a chlorine atom, an amino, nitro, cyano group or also a methoxy group, and R1 represents an ethyl, propyl or butyl radical.

8. Use according to any one of Claims 5 to 7, characterised in that the compound (I) is selected from the group comprising
- N-[(N-1-butyl-2-pyrrolidinyl)-methyl]-1-methoxy-4-bromo-2-naphthamide, possibly in the form of chlorohydrate,
- N-[(N-ethyl-2-pyrrolidinyl)-methyl]-1-methoxy-2-naphthamide,
- 4-amino-N-[(N-ethyl-2-pyrrolidinyl)-methyl]-1-methoxy-2-naphthamide, possibly in the form of dichlorohydrate,
- 4-nitro-N-[(N-ethyl-2-pyrrolidinyl)-methyl]-1-methoxy-2-naphthamide,
- N-[(N-ethyl-2-pyrrolidinyl)-methyl]-1,4-dimethoxy-2-naphthamide, possibly in the form of chlorohydrate,
- 4-chloro-N-[(N-ethyl-2-pyrrolidinyl)-methyl]-1-methoxy-2-naphthamide, possibly in the form of chlorohydrate,
- 4-bromo-N-[(N-ethyl-2-pyrrolidinyl)-methyl]-1-methoxy-2-naphthamide, possibly in the form of chlorohydrate,
- 4-methane sulphonyl amino-N-[(N-ethyl-2-pyrrolidinyl)-methyl]-1-methoxy-2-naphthamide, possibly in the form of chlorohydrate,
- 4-chloro-N-[(N-propyl-2-pyrrolidinyl)-methyl]-1-methoxy-2-naphthamide,
- 4-cyano-N-[(N-ethyl-2-pyrrolidinyl)-methyl]-1-methoxy-2-naphthamide,
- 4-cyano-N-[(N-butyl-2-pyrrolidinyl)-methyl]-1-methoxy-2-naphthamide,
- 4-cyano-N-[(N-pentyl-2-pyrrolidinyl)-methyl]-1-methoxy-2-naphthamide,
- 1,5-dimethoxy-N-[(N-ethyl-2-pyrrolidinyl)-methyl]-2-naphthamide chlorohydrate,
- 1,5-dimethoxy-N-[(N-butyl-2-pyrrolidinyl)-methyl]-2-naphthamide chlorohydrate,
- 4-bromo-N[(N,N-diethyl amino)-ethyl]-1-methoxy-2-naphthamide,
- 4-bromo-N[(morpholino)ethyl]-1-methoxy-2-naphthamide,
- 4-bromo-N[(N-butyl)-4'-piperidinyl]-1-methoxy-2-naphthamide,
- 4-bromo-N[(N-benzyl)-4'-piperidinyl]-1-methoxy-2-naphthamide,
- 4-nitro-N[(morpholino)ethyl]-1-methoxy-2-naphthamide,
- 4-nitro-N[(N-butyl-2-pyrrolidinyl)-methyl]-1,5-dimethoxy-2-naphthamide chlorohydrate,
- 4-nitro-N[(N-butyl)-4'piperidinyl]-1-methoxy-2-naphthamide,
- 4-nitro-N[(N,N-diethyl amino)-ethyl]-1-methoxy-2-naphthamide,
- 4-bromo-N[(N,N-dibutyl amino)-ethyl]-1-methoxy-2-naphthamide chlorohydrate,
- 4-bromo-N[(N-ethyl-2-pyrrolidinyl)-methyl]-1-butoxy-2-naphthamide,
- 4-nitro-N[(N-ethyl-2-pyrrolidinyl)-methyl]-1,5-dinitro-2-naphthamide,
- 4-bromo-N[(N-butyl)-4'piperidinyl]-l-allyloxy-2-naphthamide,
- 4-bromo-N[(morpholino)ethyl]-1-allyloxy-2-naphthamide chlorohydrate,
- 1,4 dimethoxy-N[(N-butyl)-4'piperidinyl]-2-naphthamide chlorohydrate,
- 4-bromo-N[(morpholino)ethyl]-1-butoxy-2-naphthamide chlorohydrate,
- 4-bromo-N[(N-ethyl-2-pyrrolidinyl)-methyl]-1-cyclopentyl-oxy-2-naphthamide chlorohydrate,
- 4-nitro-N[(N,N-dibutyl amino)-ethyl]-1-methoxy-2-naphthamide chlorohydrate,
- 4-bromo-N[(2-pyrrolidinyl)-ethyl]-1-methoxy-2-naphthamide,
- 4-cyano-N[(N,N-dibutyl amino)-ethyl]-1-methoxy-2-naphthamide chlorohydrate,
- 4-cyano-N[(N,N-dibutyl amino)-ethyl]-1-methoxy-2-naphthamide chlorohydrate.
